# EUROPEAN PATENT SPECIFICATION

(11) **EP 2 689 034 B1**
(45) Date of publication and mention of the grant of the patent: **23.03.2016**
(21) Application number: 12764214.8
(22) Date of filing: 26.03.2012
(51) Int. Cl.: C12Q 1/68, G01N 33/50, C07H 21/04

(54) **ROLE OF IFNG METHYLATION IN INFLAMMATORY BOWEL DISEASE**
ROLLE DER IFNG-METHYLIERUNG BEI ENTZÜNDLICHEN DARMERKRANKUNGEN
RÔLE DE LA MÉTHYLATION DE L'INTERFÉRON GAMMA DANS LA MALADIE INTESTINALE INFLAMMATOIRE

(30) Priority: 25.03.2011 US 201161467899 P
(43) Date of publication of application: 29.01.2014
(73) Proprietor: Cedars-Sinai Medical Center, Los Angeles, CA 90048 (US)
(72) Inventor: GONSKY, Rebecca, Los Angeles, CA 90036 (US); DEEM, Richard, Azusa, CA 91702-1509 (US); TARGAN, Stephan R., Santa Monica, CA 90402 (US)
(74) Representative: Banford, Paul Clifford
(86) International application number: PCT/US2012/030616
(87) International publication number: WO 2012/135146

(56) References cited:
- WO-A1-2007/140625
- WO-A1-2007/140625
- US-A1- 2010 041 600
- US-A1- 2011 045 476
- US-A1- 2011 045 476
- GONSKY RIVKAH ET AL: "Distinct Methylation of IFNG in the Gut.", JOURNAL OF INTERFERON & CYTOKINE RESEARCH : THE OFFICIAL JOURNAL OF THE INTERNATIONAL SOCIETY FOR INTERFERON AND CYTOKINE RESEARCH JUL 2009, vol. 29, no. 7, July 2009 (2009-07), pages 407-414, XP002731904, ISSN: 1557-7465
- RUEDA ET AL.: 'A functional variant of IFN.gamma. gene is associated with coeliac disease' GENES AND IMMUNITY vol. 5, 2004, pages 517 - 519, XP055135688
- DATABASE SNP [Online] NCBI 02 January 2001 XP055144315 Database accession no. rs1861494

## Description

### FIELD OF INVENTION

The invention relates to the field of genetics and medicine. More specifically, the invention relates to methods of diagnosing and treating inflammatory bowel disease including ulcerative colitis and Crohn's disease.

### BACKGROUND

It is not an admission that any of the information provided herein is prior art or relevant to the presently claimed invention, or that any publication specifically or implicitly referenced is prior art.

Crohn's disease (CD) and ulcerative colitis (UC), the two common forms of idiopathic inflammatory bowel disease (IBD), are chronic, relapsing inflammatory disorders of the gastrointestinal tract. Each has a peak age of onset in the second to fourth decades of life and prevalences in European ancestry populations that average approximately 100-150 per 100,000 (D.K. Podolsky, N Engl J Med 347, 417 (2002); E.V. Loftus, Jr., Gastroenterology 126, 1504 (2004)). Although the precise etiology of IBD remains to be elucidated, a widely accepted hypothesis is that ubiquitous, commensal intestinal bacteria trigger an inappropriate, overactive, and ongoing mucosal immune response that mediates intestinal tissue damage in genetically susceptible individuals (D.K. Podolsky, N Engl J Med 347, 417 (2002)). Genetic factors play an important role in IBD pathogenesis, as evidenced by the increased rates of IBD in Ashkenazi Jews, familial aggregation of IBD, and increased concordance for IBD in monozygotic compared to dizygotic twin pairs (S. Vermeire, P. Rutgeerts, Genes Immun 6, 637 (2005)). Moreover, genetic analyses have linked IBD to specific genetic variants, especially CARD 15 variants on chromosome 16q12 and the IBD5 haplotype (spanning the organic cation transporters, SLC22A4 and SLC22A5, and other genes) on chromosome 5q31 (S. Vermeire, P. Rutgeerts, Genes Immun 6, 637 (2005); J.P. Hugot et al., Nature 411, 599 (2001); Y. Ogura et al., Nature 411, 603 (2001); J.D. Rioux et al., Nat Genet 29, 223 (2001); V.D. Peltekova et al., Nat Genet 36, 471 (2004)). CD and UC are thought to be related disorders that share some genetic susceptibility loci but differ at others.

### SUMMARY

There is disclosed a method of diagnosing susceptibility to an inflammatory bowel disease (IBD) subtype in an individual, comprising obtaining a sample from the individual, assaying the sample to determine the presence or absence of at least one risk genetic variant at the genetic locus of IFNG, diagnosing susceptibility to the IBD subtype based on the presence of at least one risk genetic risk variant at the genetic locus of IFNG. In another embodiment, the IBD is ulcerative colitis. In another embodiment, the IBD is associated with early surgical intervention. In an embodiment, the IBD is associated with colitis, a small bowel disease phenotype, an aggressive complicating phenotype, an internal penetrating disease phenotype, a stricturing disease phenotype, a fibrostenosing disease phenotype, or a fistulating disease phenotype, or a combination thereof. In another embodiment, the IBD is associated with at least one risk serological marker selected from the group consisting of ANCA, ASCA, anti-Cbir1, anti-I2, and anti-OmpC. In another embodiment, the at least one risk genetic variant is a "T" allele of SEQ. ID. NO.: 1. In another embodiment, the at least one risk genetic variant is associated with a lower level of IFNG DNA methylation relative to a healthy subject. In another embodiment, the at least one risk genetic variant is associated with a higher level of anti-Cbir1 relative to a healthy subject. In another embodiment, the at least one risk genetic variant is a "C" allele of SEQ. ID. NO.: 1. In another embodiment, the at least one risk genetic variant is associated with a higher level of IFNG DNA methylation relative to a healthy subject.

Other embodiments include a method of diagnosing inflammatory bowel disease (IBD) in an individual, comprising obtaining a sample from an individual, assaying the sample to determine the presence or absence of at least one risk genetic variant at the genetic locus of IFNG, assaying the sample to determine an increase or decrease in IFNG DNA methylation relative to a healthy subject, and diagnosing IBD in the individual based on the presence of at least one risk genetic variant at the genetic locus of IFNG and an increase in IFNG DNA methylation relative to a healthy subject. In another embodiment, the IBD is Crohn's disease or ulcerative colitis. In another embodiment, the at least one risk genetic variant is a "T" allele of SEQ. ID. NO.: 1. In another embodiment, the method further comprises determining the presence of a high level of anti-Cbir1 relative to a healthy subject. In another embodiment, the IBD is associated with severe ulcerative colitis conditions. In another embodiment, the IBD is associated with colitis, a small bowel disease phenotype, an aggressive complicating phenotype, an internal penetrating disease phenotype, a stricturing disease phenotype, a fibrostenosing disease phenotype, or a fistulating disease phenotype, or a combination thereof. In another embodiment, the IBD is associated with at least one risk serological marker selected from the group consisting of ANCA, ASCA, anti-Cbir1, anti-I2, and anti-OmpC. In another embodiment, the sample comprises a nucleic acid from the individual. In another embodiment, the sample is a body fluid. In another embodiment, the body fluid is whole blood, plasma, saliva, mucus, or cheek swab. In another embodiment, the sample is a cell or tissue. In another embodiment, the cell, wherein the cell is a lymphoblastoid cell line obtained from the individual and transformed with an Epstein Barr virus. In another embodiment, the cell is a mucosal T cell, a lamina propria T cell, or a peripheral blood T cell.

Other embodiments include a method of treating an inflammatory bowel disease (IBD) in an individual, comprising obtaining a sample from an individual, assaying the sample to determine the presence of at least one risk genetic variant at the genetic locus of IFNG, assaying the sample to determine an aberrant level of IFNG DNA methylation, and treating the IBD in the individual. In another embodiment, the IBD is Crohn's disease or ulcerative colitis. In another embodiment, the IBD is associated with early surgical intervention. In another embodiment, the IBD is associated with colitis, a small bowel disease phenotype, an aggressive complicating phenotype, an internal penetrating disease phenotype, a stricturing disease phenotype, a fibrostenosing disease phenotype, or a fistulating disease phenotype, or a combination thereof. In another embodiment, the at least one risk genetic variant at the genetic locus of IFNG is SEQ. ID. NO.: 1.

Other features and advantages of the invention will become apparent from the following detailed description, taken in conjunction with the accompanying drawings, which illustrate, by way of example, various embodiments of the invention.

### BRIEF DESCRIPTION OF THE FIGURES

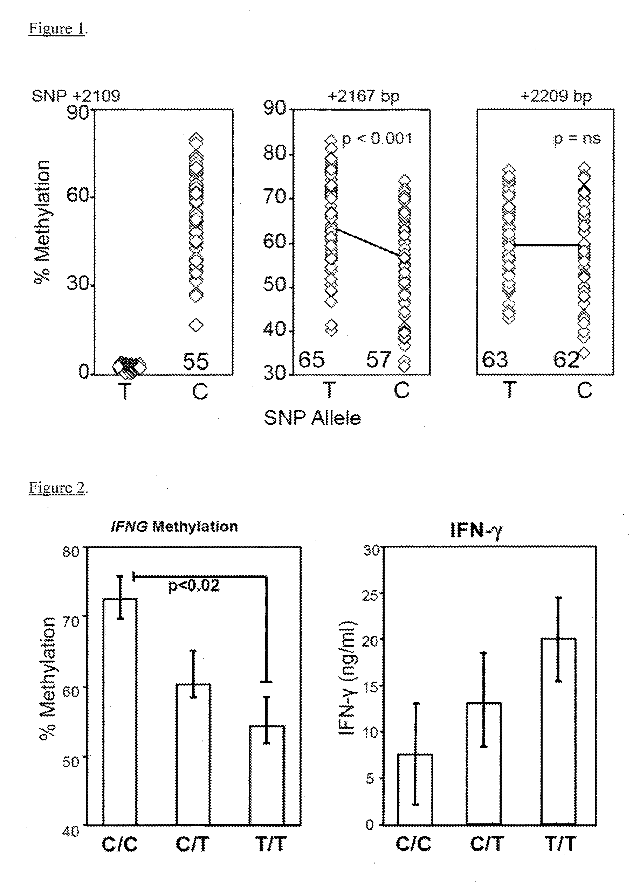

Figure 1 depicts allele specific differential methylation associated with the +2167 but not +2209 CpG site.
Figure 2 depicts IFNG SNP is functionally associated with enhanced promoter methylation and decreased protein expression.

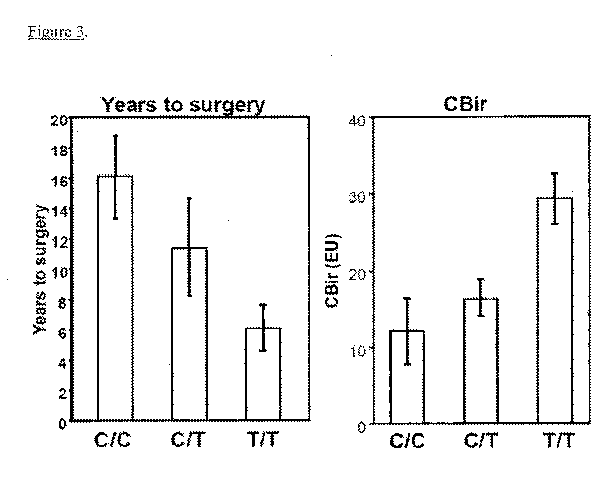

Figure 3 depicts IFNG SNP is associated with increased time to surgery and decreased Cbir responsiveness.

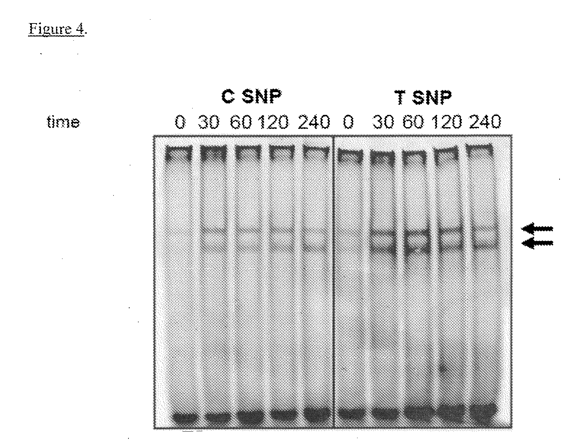

Figure 4 depicts enhanced nucleoprotein binding to IFNG rs1861494 "T" allele compared to "C" allele.

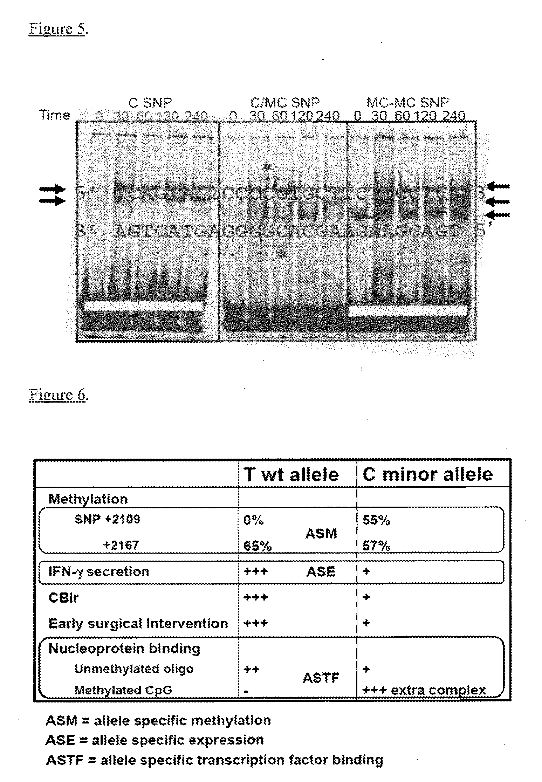

Figure 5 depicts enhanced nucleoprotein binding methylated CpG.
Figure 6 depicts a chart summarizing the IFNG research findings.

### DESCRIPTION OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 3rd ed., J. Wiley & Sons (New York, NY 2001); March, Advanced Organic Chemistry Reactions, Mechanisms and Structure 5th ed., J. Wiley & Sons (New York, NY 2001); and Sambrook and Russel, Molecular Cloning: A Laboratory Manual 3rd ed., Cold Spring Harbor Laboratory Press (Cold Spring Harbor, NY 2001), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described.

"IBD" as used herein is an abbreviation of inflammatory bowel disease.

"CD" as used herein is an abbreviation of Crohn's Disease.

"SNP" as used herein is an abbreviation of single nucleotide polymorphism.

As used herein, the term "IFNG" refers to the gene encoding IFN-gamma. Similarly, "IFNG production," or "IFNG secretion" refers to the product expressed from the IFNG genetic locus.

An example of SNP rs1861494 is provided herein as SEQ. ID. NO.: 1.

As used herein, the term "biological sample" means any biological material from which nucleic acid molecules can be prepared. As non-limiting examples, the term material encompasses whole blood, plasma, saliva, cheek swab, or other bodily fluid or tissue that contains nucleic acid.

As disclosed herein, the inventors determined what was the methylation status for *IFNG* rs1861494 SNP alleles and whether a functional relationship exists between allele specific methylation and gene expression. 154 IBD patients were genotyped for the *IFNG* rs1861494. DNA strand specific methylation levels for SNP +2109 and adjacent +2167 and +2209 CpG sites were determined by pyrosequencing. Allele and methylation-specific nucleo-protein binding was determined by EMSA. Levels of IFNG secretion and immune response to CBir were measured by ELISA.

As further disclosed herein, the wt rs1861494 T allele is un-methylated whereas the C allele displays 55% methylation. In adjacent CpG sites allele-specific DNA methylation was noted at the +2167, but not +2209, with decreased methylation of the C vs. T SNP allele DNA strands (p<0.001). The rs1861494 IFNG polymorphism is functionally associated with decreased IFNG production and levels of immune response to CBir. Allele-specific and methylation-sensitive alteration in DNA trans-factor binding patterns to the SNP was noted. Nucleo-protein binding to the unmethylated C SNP was lower than that seen for T SNP. However, methylation of the C allele strand markedly enhanced binding and the appearance of an additional nucleo-protein complex. These results link the same cis-regulatory *IFNG* variant with modulation of DNA strand methylation and transcription factor binding supporting a functional role for rs1861494 gene variant in regulating *IFNG* expression.

There is disclosed a method of diagnosing susceptibility to inflammatory bowel disease (IBD) in an individual by obtaining a sample from the individual, assaying the sample to determine the presence or absence of one or more risk genetic variants and/or an increase in IFNG DNA methylation relative to a normal subject, and diagnosing susceptiblity to inflammatory bowel disease based on the presence of one or more risk genetic variants and/or an increase in IFNG DNA methylation relative to a normal subject. In another embodiment, the IBD is ulcerative colitis. In another embodiment, the one ore more risk genetic variants include SNP rs1861494 with a "C'' allele. In another embodiment, the presence of one or more risk genetic variants and/or increase in IFNG DNA methylation relative to a normal subject is associated with a decrease in levels of IFNG expressed relative to levels found in a healthy person.

In one embodiment, the present invention provides a method of diagnosing susceptibility to inflammatory bowel disease (IBD) in an individual by assaying the sample to determine the presence or absence of one or more risk genetic variants and/or a decrease in IFNG DNA methylation relative to a normal subject, and diagnosing susceptiblity to inflammatory bowel disease based on the presence of one or more risk genetic variants which is associated with a decrease in IFNG DNA methylation relative to a normal subject. In another embodiment, the IBD is ulcerative colitis. In another embodiment, the one or more risk genetic variants include SNP rs1861494 with a "T" allele. In another embodiment, the presence of one or more risk genetic variants and/or decrease in IFNG DNA methylation relative to a normal subject is associated with a increase in levels of IFNG protein relative to levels found in a healthy person.

There is disclosed a method of treating IBD in an individual by determining the presence of aberrant DNA methylation patters at the IFNG genetic locus, relative to a healthy subject, and treating the individual.

A variety of methods can be used to determine the presence or absence of a variant allele or haplotype. As an example, enzymatic amplification of nucleic acid from an individual may be used to obtain nucleic acid for subsequent analysis. The presence or absence of a variant allele or haplotype may also be determined directly from the individual's nucleic acid without enzymatic amplification.

Analysis of the nucleic acid from an individual, whether amplified or not, may be performed using any of various techniques. Useful techniques include, without limitation, polymerase chain reaction based analysis, sequence analysis and electrophoretic analysis. As used herein, the term "nucleic acid" means a polynucleotide such as a single or double-stranded DNA or RNA molecule including, for example, genomic DNA, cDNA and mRNA. The term nucleic acid encompasses nucleic acid molecules of both natural and synthetic origin as well as molecules of linear, circular or branched configuration representing either the sense or antisense strand, or both, of a native nucleic acid molecule.

The presence or absence of a variant allele or haplotype may involve amplification of an individual's nucleic acid by the polymerase chain reaction. Use of the polymerase chain reaction for the amplification of nucleic acids is well known in the art (see, for example, Mullis et al. (Eds.), The Polymerase Chain Reaction, Birkhauser, Boston, (1994)).

A TaqmanB allelic discrimination assay available from Applied Biosystems may be useful for determining the presence or absence of a variant allele. In a TaqmanB allelic discrimination assay, a specific, fluorescent, dye-labeled probe for each allele is constructed. The probes contain different fluorescent reporter dyes such as FAM and VICTM to differentiate the amplification of each allele. In addition, each probe has a quencher dye at one end which quenches fluorescence by fluorescence resonant energy transfer (FRET). During PCR, each probe anneals specifically to complementary sequences in the nucleic acid from the individual. The 5' nuclease activity of Taq polymerase is used to cleave only probe that hybridize to the allele. Cleavage separates the reporter dye from the quencher dye, resulting in increased fluorescence by the reporter dye. Thus, the fluorescence signal generated by PCR amplification indicates which alleles are present in the sample. Mismatches between a probe and allele reduce the efficiency of both probe hybridization and cleavage by Taq polymerase, resulting in little to no fluorescent signal. Improved specificity in allelic discrimination assays can be achieved by conjugating a DNA minor grove binder (MGB) group to a DNA probe as described, for example, in Kutyavin et al., "3'-minor groove binder-DNA probes increase sequence specificity at PCR extension temperature, "Nucleic Acids Research 28:655-661 (2000)). Minor grove binders include, but are not limited to, compounds such as dihydrocyclopyrroloindole tripeptide (DPI,).

Sequence analysis also may also be useful for determining the presence or absence of a variant allele or haplotype.

Restriction fragment length polymorphism (RFLP) analysis may also be useful for determining the presence or absence of a particular allele (Jarcho et al. in Dracopoli et al., Current Protocols in Human Genetics pages 2.7.1-2.7.5, John Wiley & Sons, New York; Innis et al.,(Ed.), PCR Protocols, San Diego: Academic Press, Inc. (1990)). As used herein, restriction fragment length polymorphism analysis is any method for distinguishing genetic polymorphisms using a restriction enzyme, which is an endonuclease that catalyzes the degradation of nucleic acid and recognizes a specific base sequence, generally a palindrome or inverted repeat. One skilled in the art understands that the use of RFLP analysis depends upon an enzyme that can differentiate two alleles at a polymorphic site.

Allele-specific oligonucleotide hybridization may also be used to detect a disease-predisposing allele. Allele-specific oligonucleotide hybridization is based on the use of a labeled oligonucleotide probe having a sequence perfectly complementary, for example, to the sequence encompassing a disease-predisposing allele. Under appropriate conditions, the allele-specific probe hybridizes to a nucleic acid containing the disease-predisposing allele but does not hybridize to the one or more other alleles, which have one or more nucleotide mismatches as compared to the probe. If desired, a second allele-specific oligonucleotide probe that matches an alternate allele also can be used. Similarly, the technique of allele-specific oligonucleotide amplification can be used to selectively amplify, for example, a disease-predisposing allele by using an allele-specific oligonucleotide primer that is perfectly complementary to the nucleotide sequence of the disease-predisposing allele but which has one or more mismatches as compared to other alleles (Mullis et al., supra, (1994)). One skilled in the art understands that the one or more nucleotide mismatches that distinguish between the disease-predisposing allele and one or more other alleles are preferably located in the center of an allele-specific oligonucleotide primer to be used in allele-specific oligonucleotide hybridization. In contrast, an allele-specific oligonucleotide primer to be used in PCR amplification preferably contains the one or more nucleotide mismatches that distinguish between the disease-associated and other alleles at the 3' end of the primer.

A heteroduplex mobility assay (HMA) is another well known assay that may be used to detect a SNP or a haplotype. HMA is useful for detecting the presence of a polymorphic sequence since a DNA duplex carrying a mismatch has reduced mobility in a polyacrylamide gel compared to the mobility of a perfectly base-paired duplex (Delwart et al., Science 262:1257-1261 (1993); White et al., Genomics 12:301-306 (1992)).

The technique of single strand conformational, polymorphism (SSCP) also may be used to detect the presence or absence of a SNP and/or a haplotype (see Hayashi, K., Methods Applic. 1:34-38 (1991)). This technique can be used to detect mutations based on differences in the secondary structure of single-strand DNA that produce an altered electrophoretic mobility upon non-denaturing gel electrophoresis. Polymorphic fragments are detected by comparison of the electrophoretic pattern of the test fragment to corresponding standard fragments containing known alleles.

Denaturing gradient gel electrophoresis (DGGE) also may be used to detect a SNP and/or a haplotype. In DGGE, double-stranded DNA is electrophoresed in a gel containing an increasing concentration of denaturant; double-stranded fragments made up of mismatched alleles have segments that melt more rapidly, causing such fragments to migrate differently as compared to perfectly complementary sequences (Sheffield et al., "Identifying DNA Polymorphisms by Denaturing Gradient Gel Electrophoresis" in Innis et al., supra, 1990).

Other molecular methods useful for determining the presence or absence of a SNP and/or a haplotype are known in the art and useful in the methods of the invention. Other well-known approaches for determining the presence or absence of a SNP and/or a haplotype include automated sequencing and RNAase mismatch techniques (Winter et al., Proc. Natl. Acad. Sci. 82:7575-7579 (1985)). Furthermore, one skilled in the art understands that, where the presence or absence of multiple alleles or haplotype(s) is to be determined, individual alleles can be detected by any combination of molecular methods. See, in general, Birren et al. (Eds.) Genome Analysis: A Laboratory Manual Volume 1 (Analyzing DNA) New York, Cold Spring Harbor Laboratory Press (1997). In addition, one skilled in the art understands that multiple alleles can be detected in individual reactions or in a single reaction (a "multiplex" assay). In view of the above, one skilled in the art realizes that the methods of the present invention for diagnosing or predicting susceptibility to or protection against CD in an individual may be practiced using one or any combination of the well known assays described above or another art-recognized genetic assay.

One skilled in the art will recognize many methods and materials similar or equivalent to those described herein, which could be used in the practice of the present invention. Indeed, the present invention is in no way limited to the methods and materials described.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### Example 1

Epigenetic remodeling of chromatin via DNA methylation affects transcriptional activation. It has been demonstrated a distinct *IFNG* DNA methylation pattern in mucosal T cells from IBD patients and in peripheral T cells of a subset of UC patients. Decreased *IFNG* methylation was associated with increased IFNG production and seroreactivity to microbial antigens. GWA Studies identified UC-risk/severity regions linked to single nucleotide polymorphisms (SNP) flanking *IFNG.* One of the challenges of GWAS is to define the functional consequences of these genetic variations. Many disease-associated SNPs target CpG sites, which are relatively rare within the genome and serve as sites for DNA methylation. Recently, allele specific methylation was reported to preferentially occur at CpG sites adjacent to SNPs that alter CpG sites. The CpG (C/T) SNP rs1861494 (+2109) is located in a conserved regulatory region of the third intron of *IFNG,* within the same LD block implicated with UC and disease severity. Two adjacent CpG sites are found at +2167 and +2209 bp. Though typically both alleles contribute towards gene expression, monoallelic expression of IFNG protein has been reported. Moreover, it seems likely that variants that alter CpG sites not only alter methylation but may lead to unequal allelic expression.

The inventors determined what was the methylation status for *IFNG* rs1861494 SNP alleles and whether a functional relationship exists between allele specific methylation and gene expression. 154 IBD patients were genotyped for the *IFNG* rs1861494. DNA strand specific methylation levels for SNP +2109 and adjacent +2167 and +2209 CpG sites were determined by pyrosequencing. Allele and methylation-specific nucleo-protein binding was determined by EMSA. Levels of IFNG secretion and immune response to CBir were measured by ELISA.

The wt rs1861494 T allele is un-methylated whereas the C allele displays 55% methylation. In adjacent CpG sites allele-specific DNA methylation was noted at the +2167, but not +2209, with decreased methylation of the C vs. T SNP allele DNA strands (p<0.001). The rs1861494 IFNG polymorphism is functionally associated with decreased IFNG production and levels of immune response to CBir. Allele-specific and methylation-sensitive alteration in DNA trans-factor binding patterns to the SNP was noted. Nucleo-protein binding to the unmethylated C SNP was lower than that seen for T SNP. However, methylation of the C allele strand markedly enhanced binding and the appearance of an additional nucleo-protein complex. These results link the same cis-regulatory *IFNG* variant with modulation of DNA strand methylation and transcription factor binding supporting a functional role for rs1861494 gene variant in regulating *IFNG* expression.

While the description above refers to particular embodiments of the present invention, it should be readily apparent to people of ordinary skill in the art that a number of modifications may be made without departing from the spirit thereof. The presently disclosed embodiments are, therefore, to be considered in all respects as illustrative and not restrictive.

The various methods and techniques described above provide a number of ways to carry out the invention. Of course, it is to be understood that not necessarily all objectives or advantages described may be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as may be taught or suggested herein. A variety of advantageous and disadvantageous alternatives are mentioned herein. It is to be understood that some preferred embodiments specifically include one, another, or several advantageous features, while others specifically exclude one, another, or several disadvantageous features, while still others specifically mitigate a present disadvantageous feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be mixed and matched by one of ordinary skill in this art to perform methods in accordance with principles described herein. Among the various elements, features, and steps some will be specifically included and others specifically excluded in diverse embodiments.

Although the invention has been disclosed in the context of certain embodiments and examples, it will be understood by those skilled in the art that the embodiments of the invention extend beyond the specifically disclosed embodiments to other alternative embodiments and/or uses and modifications and equivalents thereof.

Many variations and alternative elements have been disclosed in embodiments of the present invention. Still further variations and alternate elements will be apparent to one of skill in the art. Among these variations, without limitation, are the selection of constituent modules for the inventive compositions, and the diseases and other clinical conditions that may be diagnosed, prognosed or treated therewith. Various embodiments of the invention can specifically include or exclude any of these variations or elements.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventors for carrying out the invention. Variations on those preferred embodiments will become apparent to those of ordinary skill in the art upon reading the foregoing description. It is contemplated that skilled artisans can employ such variations as appropriate, and the invention can be practiced otherwise than specifically described herein. Accordingly, many embodiments of this invention include all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification.

In closing, it is to be understood that the embodiments of the invention disclosed herein are illustrative of the principles of the present invention. Other modifications that can be employed can be within the scope of the invention. Thus, by way of example, but not of limitation, alternative configurations of the present invention can be utilized in accordance with the teachings herein. Accordingly, embodiments of the present invention are not limited to that precisely as shown and described.

## Claims

1. A method of diagnosing susceptibility to an inflammatory bowel disease (IBD) subtype in an individual, comprising:
(a) assaying a sample obtained from the individual to determine the presence or absence of at least one risk genetic variant at the genetic locus of IFNG; and
(b) diagnosing susceptibility to the IBD subtype based on the presence of at least one genetic risk variant at the genetic locus of IFNG
wherein the at least one risk genetic variant is a "T" allele of SEQ. ID. NO.: 1 which is associated with a lower level of IFNG DNA methylation relative to a healthy subject.

2. The method of claim 1, wherein the IBD comprises ulcerative colitis.

3. The method of claim 1 wherein the IBD is associated with early surgical intervention.

4. The method of claim 1, wherein the at least one risk genetic variant is associated with a higher level of anti-Cbir1 relative to a healthy subject.

5. A method of diagnosing inflammatory bowel disease (IBD) in an individual, comprising:
(a) assaying a sample obtained from the individual to determine the presence or absence of at least one risk genetic variant at the genetic locus of IFNG;
(b) assaying the sample to determine an increase or decrease in IFNG DNA methylation relative to a healthy subject;
(c) diagnosing IBD in the individual based on the presence of a risk genetic variant which is a "T" allele of SEQ. ID. NO.: 1 and which is associated with a lower level of IFNG DNA methylation relative to a healthy subject.

6. The method of claim 5 wherein the IBD comprises Crohn's disease or ulcerative colitis.

7. The method of claim 5, further comprising assaying the sample to identify a high level of anti-Cbir1 relative to a healthy subject.

8. The method of claim 5, wherein the IBD is associated with severe ulcerative colitis conditions.

9. The method of claim 1 or 5 wherein the IBD is associated with colitis, a small bowel disease phenotype, an aggressive complicating phenotype, an internal penetrating disease phenotype, a stricturing disease phenotype, a fibrostenosing disease phenotype, a fistulating disease phenotype, or a combination thereof.

10. The method of claim 1 or 5, wherein the IBD is associated with at least one risk serological marker selected from the group consisting of ANCA, ASCA, anti-Cbir1, anti-I2, and anti-OmpC.

11. The method of claim 5, wherein the sample comprises a nucleic acid from the individual.

12. The method of claim 5, wherein the sample is a body fluid and may optionally be selected from whole blood, plasma, saliva, mucus, or cheek swab.

13. The method of claim 5, wherein the sample is a cell or tissue.

14. The method of claim 13, wherein the cell is a lymphoblastoid cell line obtained from the individual and transformed with an Epstein Barr virus; a mucosal T cell; a lamina propria T cell; or a peripheral blood T cell.

## Patentansprüche

1. Verfahren zur Diagnose der Anfälligkeit für einen Subtyp einer chronisch entzündlichen Darmerkrankung (CED) in einem Individuum, umfassend:
(a) Untersuchen einer von dem Individuum erhaltenen Probe, um das Vorhandensein oder die Abwesenheit wenigstens einer genetischen Risikovariante an dem Genlokus von IFNG zu bestimmen; und
(b) Diagnostizieren des CED-Subtyps auf der Basis des Vorhandenseins wenigstens einer genetischen Risikovariante an dem Genlokus von IFNG;
wobei die wenigstens eine genetische Risikovariante ein "T"-Allel der SEQ ID NO.: 1 ist, das einem niedrigeren Wert der IFNG-DNA-Methylierung im Verhältnis zu einem gesunden Subjekt assoziiert ist.

2. Verfahren nach Anspruch 1, wobei die CED Colitis ulcerosa ist.

3. Verfahren nach Anspruch 1, wobei die CED einem frühzeitigen chirurgischen Eingriff assoziiert ist.

4. Verfahren nach Anspruch 1, wobei die wenigstens eine genetische Risikovariante einem höheren Wert von Anti-CBir1 im Verhältnis zu einem gesunden Subjekt assoziiert ist.

5. Verfahren zur Diagnose einer chronisch entzündlichen Darmerkrankung (CED) in einem Individuum, umfassend:
(a) Untersuchen einer von dem Individuum erhaltenen Probe, um das Vorhandensein oder die Abwesenheit wenigstens einer genetischen Risikovariante an dem Genlokus von IFNG zu bestimmen;
(b) Untersuchen der Probe, um einen Anstieg oder Rückgang der IFNG-DNA-Methylierung im Verhältnis zu einem gesunden Subjekt zu bestimmen; und
(c) Diagnostizieren der CED in dem Individuum auf der Basis des Vorhandenseins einer genetischen Risikovariante, die ein "T"-Allel der SEQ ID NO.: 1 ist, und die einem niedrigeren Wert der IFNG-DNA-Methylierung im Verhältnis zu einem gesunden Subjekt assoziiert ist.

6. Verfahren nach Anspruch 5, wobei die CED die Crohnsche Krankheit oder Colitis ulcerosa umfasst.

7. Verfahren nach Anspruch 5, ferner umfassend das Untersuchen der Probe, um einen hohen Wert von Anti-CBir1 im Verhältnis zu einem gesunden Subjekt zu identifizieren.

8. Verfahren nach Anspruch 5, wobei die CED mit schweren Zuständen von Colitis ulcerosa assoziiert ist.

9. Verfahren nach einem der Ansprüche 1 bis 5, wobei die CED Colitis, dem Phänotyp Dünndarmkrankheit, einem aggressiven, erschwerenden Phänotyp, einem Phänotyp einer inneren penetrierenden Erkrankung, einem Phänotyp einer Stenose, einem Phänotyp einer Fibrostenose, einem Phänotyp einer fistelbildenden Erkrankung oder einer Kombination davon assoziiert ist.

10. Verfahren nach Anspruch 1 oder 5, wobei die CED wenigstens einem risikoserologischen Marker assoziiert ist, der ausgewählt ist aus der Gruppe bestehend aus ANCA, ASCA, Anti-CBir1, Anti-12 und Anti-OmpC.

11. Verfahren nach Anspruch 5, wobei die Probe eine Nukleinsäure des Individuums umfasst.

12. Verfahren nach Anspruch 5, wobei die Probe ein Körperfluid ist und optional ausgewählt sein kann aus Vollblut, Plasma, Speichel, Schleim oder einem Wangenabstrich.

13. Verfahren nach Anspruch 5, wobei die Probe eine Zelle oder Gewebe ist.

14. Verfahren nach Anspruch 13, wobei die Zelle eine lymphoblastische Zelllinie ist, erhalten von dem Individuum und transformiert mit einem Epstein-Bar-Virus, eine mukosale T-Zelle, eine Lamina propria T-Zelle oder eine T-Zelle von peripherem Blut.

## Revendications

1. Procédé pour le diagnostic de la susceptibilité à un sous-type de la maladie inflammatoire chronique de l'intestin (IBD) chez un individu, comprenant les étapes consistant à :
(a) titrer un échantillon obtenu à partir de l'individu pour déterminer la présence ou l'absence d'au moins une variante d'un risque génétique au locus génétique de l'IFNG ; et
(b) diagnostiquer la susceptibilité au sous-type IBD sur la base de la présence d'au moins une variante d'un risque génétique au locus génétique de l'IFNG
dans lequel l'au moins une variante d'un risque génétique est une allèle « T » de SEQ. ID. NO.: 1 qui est associée à un niveau inférieur de la méthylation de l'ADN de l'IFNG par rapport à un sujet sain.

2. Procédé selon la revendication 1, dans lequel l'IBD comprend la colite ulcéreuse.

3. Procédé selon la revendication 1, dans lequel l'IBD est associée à une intervention chirurgicale précoce.

4. Procédé selon la revendication 1, dans lequel l'au moins une variante d'un risque génétique est associée à un niveau supérieur d'anti-Cbir1 par rapport à un sujet sain.

5. Procédé pour le diagnostic de la maladie inflammatoire chronique de l'intestin (IBD) chez un individu, comprenant les étapes consistant à :
(a) titrer un échantillon obtenu à partir de l'individu pour déterminer la présence ou l'absence d'au moins une variante d'un risque génétique au locus génétique de l'IFNG ;
(b) titrer l'échantillon pour déterminer une augmentation ou diminution de la méthylation de l'ADN de l'IFNG par rapport à un sujet sain ;
(c) diagnostiquer l'IBD chez l'individu sur la base de la présence d'au moins une variante d'un risque génétique au locus génétique de l'IFNG qui est une allèle « T » de SEQ. ID. NO.: 1 et qui est associée à un niveau inférieur de la méthylation de l'ADN de l'IFNG par rapport à un sujet sain.

6. Procédé selon la revendication 5, dans lequel l'IBD comprend la maladie de Crohn ou la colite ulcéreuse.

7. Procédé selon la revendication 5, comprenant en outre l'étape consistant à titrer l'échantillon pour identifier un niveau élevé d'anti-Cbirl par rapport à un sujet sain.

8. Procédé selon la revendication 5, dans lequel l'IBD est associée à des états sévères de colite ulcéreuse.

9. Procédé selon la revendication 1 ou la revendication 5, dans lequel l'IBD est associée à la colite, au phénotype de l'intestin grêle, à un phénotype agressif compliquant, à un phénotype d'une maladie interne pénétrante, à un phénotype de la maladie sténosante, à un phénotype de la sténose fibreuse, à un phénotype de la maladie fistulante, ou une combinaison de ceux-ci.

10. Procédé selon la revendication 1 ou la revendication 5, dans lequel l'IBD est associé avec au moins un marqueur sérologique de risque choisi parmi le groupe constitué d'ANCA, d'ASCA, d'anti-Cbir1, d'anti-I2, et d'anti-OmpC.

11. Procédé selon la revendication 5, dans lequel l'échantillon comprend un acide nucléique de l'individu.

12. Procédé selon la revendication 5, dans lequel l'échantillon est un fluide corporel et peut éventuellement être choisi parmi le sang total, plasma, salive, mucus, ou frottis buccal.

13. Procédé selon la revendication 5, dans lequel l'échantillon est une cellule ou un tissu.

14. Procédé selon la revendication 13, dans lequel la cellule est une lignée cellulaire de lymphoblastoïde obtenue à partir de l'individu et transformée avec un virus d'Epstein Barr ; une cellule T de la muqueuse ; une cellule T de lamina propria ; ou une cellule T de sang périphérique.


### SEQUENCE LISTING

<110> CEDARS-SINAI MEDICAL CENTER GONSKY, Rivkah TARGAN, Stephan R.
<120> ROLE OF IFNG METHYLATION IN INFLAMMATORY BOWEL DISEASE
<130> 67789-316WO0
<150> US 61/467,899
   <151> 2011-03-25
<160> 1
<170> PatentIn version 3.5
<210> 1
   <211> 620
   <212> DNA
   <213> Homo sapiens
<400> 1
   tctgaaagtt gatagagagt tgatagaaca atcttttcat aaggtataaa ttctatctat    60
   ttttccctaa aaacaaacaa acagcaacca ttcttgcttc taattgggca gtacaatctg   120
   ataggttggc tagagacttg cagtggggtg tccctggtac ctattcaaag actgtagctt   180
   tcttctatct cattctcatt ttctattctt tgcattgtag agttttggag caaagaaggt   240
   catcaaactt atacagtgag cctaacagtt tccttttaag atgaggaaac tgagccccag   300
   ccagccatgt gattcatcac agttccttgg tggctgagtt gggaggagaa cacacatctt   360
   ctcagctcct cccactgctc tttccattaa gacagacagc ctctcattca aagtaagaga   420
   atttccatca tatgagcaag ggacaatgag agaactgctt ctcagtactc ccygcttctt   480
   cctcacctac ttcctcttca ctggatttgt caactcacct gtctttacgc aatagttaca   540
   atgccagcat ttctctacat tacatacttc agcgattctc ttactggctt tgcaaagtca   600
   cccaaacacg aatggaaata                                               620